# EUROPEAN PATENT APPLICATION

(11) **EP 4 830 714 A2**
(43) Date of publication of application: **28.10.2026**
(21) Application number: 26192788.3
(22) Date of filing: 10.05.2018
(51) Int. Cl.: A61K 38/00

(54) **ENVELOPED VIRUS RESISTANT TO COMPLEMENT INACTIVATION FOR THE TREATMENT OF CANCER**

(30) Priority: 10.05.2017 US 201762504120 P
(62) Document number(s) of the earlier application(s) in accordance with Art. 76 EPC: 18798575.9 / 3 621 635
(71) Applicant: Pharma Cinq, LLC, Rockville, MD 20850 (US)
(72) Inventor: LUO, Tianci, Fulton, MD 20759 (US); MOLINA, Rene, Hagerstown, MD 21740 (US); CASTILLE, Gabriel, Kensington, MD 20895 (US)
(74) Representative: Mathys & Squire

(57) **Abstract**

A recombinant fusion protein is disclosed. The fusion protein comprises: (a) a CD55 peptide sequence, (b) a linker sequence C-terminal to the CD55 sequence, (c) a transmembrane domain C-terminal to the linker sequence, and (d) an intracellular domain C-terminal to the transmembrane domain. The fusion protein does not contain a GPI anchor. The fusion protein can be expressed with an N-terminal secretory signal peptide, which is cleaved to yield the mature protein on the surface of a cell line or an enveloped virus. An oncolytic virus expressing the fusion protein is resistant to complement inactivation and can be used to treat cancer.

## Description

### REFERENCE TO SEQUENCE LISTING

The Sequence Listing filed electronically in the form of an Annex C/ST.25 text file and bearing file reference 21003-PCT is a part of the disclosure.

### BACKGROUND OF THE INVENTION

Oncolytic viruses have been tested as agents for the treatment of cancers by infecting and destroying tumor cells. These oncolytic viruses include Newcastle Disease Virus, Adenovirus, Sindbis virus, Vaccinia virus, Herpes virus etc. Newcastle Disease Virus (NDV) showed a great potential in shrinking tumor in cancer patients due to its unique property of preferential replication in and lysis of tumor cells, presumably owing to the factor that most tumor cells have a deficient interferon pathway (Pecora et al., 2002; Laurie et al., 2006; Lorence et al., 2007). Despite the preliminary promising clinical results, NDV as a cancer therapeutic agent has a shortcoming: inevitably most of the NDV particles will be destroyed by the patient's innate immune system, the alternative complement pathway, once the viruses enter the patient's body.

The complement system is a part of the innate and adaptive immune system (reviewed by Volanakis, J.E., 1998. Chapter 2. In The Human Complement System in Health and Disease. Edited by J. E. Volanakis, and M.M. Frank. Marcel Dekker, Inc., New York pp 9-32). Complement plays an important role in microbial killing, and for the transport and clearance of immune complexes. Many of the activation products of the complement system are also associated with proinflammatory or immunoregulatory functions. The complement system consists of plasma and membrane-associated proteins that are organized in three enzymatic-activation cascades: the classical, the lectin, and the alternative pathways. All three pathways can lead to the formation of the terminal complement complex/membrane attack complex (TCC/MAC) and an array of biologically active products.

Human cells and organs have a family of membrane-bound complement regulatory proteins to protect them from homologous complement-mediated lysis. These complement regulatory proteins include CD55 (decay-accelerating factor, DAF), CD46 (membrane cofactor protein, MCP), CD35 (complement receptor 1, CR1), and CD59 (membrane inhibitor of reactive lysis) (Carroll et al., 1988; Rey-Campos et al., 1988; Lublin et al., 1989; Morgan et al., 1994; Kim and Song, 2006).

CD55 is a glycosylphosphatidylinositol (GPI)-anchored protein and attaches to a cell plasma membrane through a glycolipid moiety (GPI anchor) at its C-terminus. The GPI-anchored proteins such as CD55 can be endocytosed and degraded or cleaved and released from cell plasma membrane (Censullo and Davitz, 1994a, 1994b; Turner 1994). For example, The GPI-anchored proteins including CD55 can be released from the cell surface by the action of GPI-specific phospholipases C and D (Turner 1994). These enzymatic activities likely control the catabolism of GPI-anchored proteins and regulate the cell surface expression of these proteins (Censullo and Davitz, 1994b).

### SUMMARY OF THE INVENTION

This invention provides a recombinant fusion protein comprising: (a) a CD55 peptide sequence, (b) a linker sequence C-terminal to the CD55 sequence, (c) a transmembrane domain C-terminal to the linker sequence, and (d) an intracellular domain C-terminal to the transmembrane domain, wherein the fusion protein does not contain a GPI anchor. This invention also provides nucleic acids and expression vectors encoding the protein, cells expressing the protein, enveloped viruses incorporating the protein on the viral membrane, pharmaceutical compositions comprising the protein-incorporating virus of this invention, as well as methods of treatment and uses of the virus.

This invention is based, in part, on the finding that virus expressing a fusion protein according to this invention was resistant to inactivation by normal human serum, as evidenced by a higher recovery rate compared to virus not expressing the fusion protein. The oncolytic enveloped virus produced by the engineered cells of the invention which incorporate complement inhibitor in the form of a recombinant fusion protein on the viral membrane is a better cancer therapeutic and affords better clinical outcomes for cancer patients as compared to the corresponding virus lacking a complement inhibitor on the viral membrane, due to its survival capability in the human serum circulation before it gets into a tumor. The benefits are three-fold: 1) the oncolytic virus can be produced in a cell culturing system in a bio-reactor; 2) fewer viral particles are needed to achieve the same therapeutic efficacy as compared to the parental oncolytic virus produced in chicken eggs; 3) infusion of fewer viral particles to a cancer patient may reduce side effects associated with large amounts of viral particles such as cytokine storm or impurity related effects.

Others who have studied the effects of the complement regulatory protein CD55 on the protection of Newcastle Disease Virus (NDV) (Biswas et al., 2012; Rangaswamy et al., 2016) used native unmodified CD55, which includes a glycosylphosphatidyl-inositol (GPI) anchor. In contrast the fusion protein of this invention omits the GPI anchor. Without wishing to be bound by theory it is believed that the omission of a GPI anchor changed the catabolism dynamics of CD55 on the cell surface. The fusion protein of this invention was able to withstand inactivation conditions more stringent than those utilized by Biswas and Rangaswamy. Biswas used 5 to 10% normal human serum and Rangaswamy used 0.3 to 5% normal human serum in their inactivation assays. The example below used 40% normal human serum to conduct the inactivation assay on NDV that has incorporated the recombinant fusion protein.

### BRIEF DESCRIPTION OF THE FIGURES

Figure 1. Mammalian cell expression construct map sequence encoding a recombinant complement inhibitory fusion protein consisting of a secretory signal peptide, four short consensus repeat (SCR) of CD55, a flexible linker, a CD8 transmembrane domain and a truncated CD8 intracellular domain, followed by IRES-neo selectable marker and a synthetic polyadenylation signal (polyA).

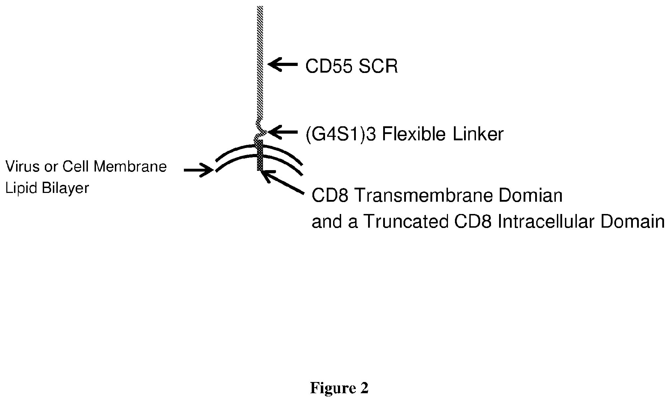

Figure 2. Diagram illustrating the orientation of the matured complement inhibitory fusion protein on the engineered DF1 cell membrane or on modified NDV membrane.
Figure 3. Cell surface expression of a recombinant complement inhibitory fusion protein. Flow cytometry analysis for the fusion protein expression by a CD55 specific antibody. The histogram on the left represents the naive DF1 cells as a negative control. The histogram on the right represents the DF1 cells stably expressing SEQ ID NO:2 (cell clone number 8).
Figure 4. Cytotoxicity assay of NDV produced by the engineered DF1 cells (Clone number 8) incorporated with the complement inhibitory fusion protein in tumor cell lines.

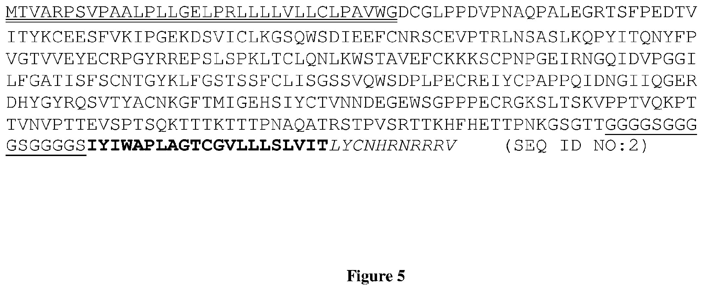

Figure 5. Amino Acid Sequence of a recombinant complement inhibitory fusion protein consisting of secretory signal peptide, four short consensus repeat (SCR) of CD55, a flexible linker, a CD8 transmembrane domain and a truncated CD8 intracellular domain. (SEQ ID NO:2)
   Double Underlined indicates Secretory signal peptide
   Regular type indicates SCR of CD55
   Underlined indicates (G4S1)3 Linker
   **Bold** indicates the CD8 transmembrane domain
   *Italic* indicates the truncated CD8 intracellular domain

### DETAILED DESCRIPTION OF THE INVENTION

In accordance with the fusion protein of this invention any CD55 peptide sequence can be utilized for sequence (a). In an embodiment the CD55 peptide sequence is a human CD55 peptide sequence. The CD55 peptide sequence preferably comprises four short consensus repeats (SCR) of CD55. Any flexible linker can bc utilized for sequence (b), for example a conventional flexible linker known in the field. In one embodiment a G4S1 linker is utilized, preferably a (G4S1)3 linker. Any transmembrane domain can be utilized for sequence (c), for example a conventional transmembrane domain known in the field. In one embodiment the transmembrane domain is a CD8 transmembrane domain. Any intracellular domain can be utilized for sequence (d), for example a conventional intracellular domain. In one embodiment the transmembrane domain is a CD8 transmembrane domain, preferably a truncated CD8 transmembrane domain.

The fusion protein of this invention can further comprise a secretory signal peptide N-terminal to sequence (a). In accordance with the preferred process of this invention the fusion protein is initially expressed with the signal peptide. The signal peptide directs the newly synthesized fusion protein to the endoplasmic reticulum (ER), where the signal peptide is cleaved by signal peptidase. SEQ ID NO:2 is an exemplary fusion protein of this invention having an N-terminal signal peptide. SEQ ID NO:3 is an exemplary fusion protein of this invention not having an N-terminal signal peptide.

In accordance with the fusion protein of this invention there can optionally be a spacer of one or more amino acids between the N-terminal signal peptide and sequence (a), between sequence (a) and sequence (b), between sequence (b) and sequence (c), between sequence (c) and sequence (d), between any two of them, between any three of them, or between all four. In one embodiment of this invention there is no spacer between the N-teminal signal peptide and sequence (a), or in other words the N-terminal signal peptide is covalently bonded to sequence (a) by a single peptide bond. In another embodiment there is a spacer between the N-terminal signal peptide and sequence (a).

In one embodiment of this invention there is no spacer between sequence (a) and sequence (b), or in other words sequence (a) is covalently bonded to sequence (b) by a single peptide bond. In another embodiment there is a spacer between sequence (a) and sequence (b). In one embodiment of this invention there is no spacer between sequence (b) and sequence (c), or in other words sequence (b) is covalently bonded to sequence (c) by a single peptide bond. In another embodiment there is a spacer between sequence (b) and sequence (c). In one embodiment of this invention there is no spacer between sequence (c) and sequence (d), or in other words sequence (c) is covalently bonded to sequence (d) by a single peptide bond. In another embodiment there is a spacer between sequence (c) and sequence (d). There is in principle no limitation on the size of the spacers.

CD55 contains four extracellular short consensus repeat (SCR), a Ser/Thr/Pro (STP)-rich region and a GPI-anchored domain. In accordance with the fusion protein of this invention the GPI-anchor domain is omitted. The STP-rich region can be present or absent. One embodiment of the fusion protein coding sequence of this invention further comprises a polyadenylation signal C-terminal to the third peptide sequence coding sequence. The polyadenylation signal (Poly A) can be any Poly A.

This invention provides a nucleic acid encoding the protein described above. In one embodiment the nucleic acid is DNA. It can optionally contain one or more introns, either between the sequences coding for the signal peptide and sequence (a), between sequence (a) and sequence (b), between sequence (b) and sequence (c), between sequence (c) and sequence (d), or elsewhere. In an embodiment of this invention the nucleic acid encodes a protein having the sequence SEQ ID NO:2 or SEQ ID NO:3. SEQ ID NO:1 is one example of a nucleic acid encoding a protein having the sequence SEQ ID NO:2. Because different nucleic acid codon triplets code for the same amino acid, a relationship known as the degeneracy of the genetic code, many other nucleic acid sequences that encode a protein having the sequence SEQ ID NO:2 can readily be envisioned and are included in this invention.

An embodiment of this invention is an expression vector comprising the nucleic acid described above operatively linked to a control sequence, for example a promoter. The promoter driving the fusion protein can be any promoter and is not limited to a CMV promoter. When there is an intron between the promoter and the fusion protein coding sequence, any suitable and conventional intron can be utilized. For example, a β-globin intron is suitable.

This invention provides a cell line stably expressing the fusion protein of this invention on its cell surface. Any conventional cell line for protein expression can be used in accordance with this invention. In one embodiment the cell line is a mammalian cell line. In another embodiment the cell line is a non-mammalian cell line, for example a DF-1 chicken embryonic fibroblast cell line.

This invention provides an enveloped virus incorporating the fusion protein described above on the virus membrane. In accordance with this invention any enveloped virus can be utilized. In an embodiment the virus is an oncolytic virus, for example a paramyxovirus such as Newcastle Disease Virus (NDV). In the examples a complement inhibitor in the form of a recombinant fusion protein was incorporated onto NDV particles envelope. The recombinant fusion protein of this invention could be used for oncolytic viruses other than NDV, leading to generation of oncolytic viral particles that are more resistant to host complement inactivation. The novel recombinant complement inhibitor in the form of a fusion protein can be used to modify any other mammalian cells such as HeLa cells to produce oncolytic viruses. Oncolytic viruses are described in International Patent Publication No. WO 2000/062735, the content of which is incorporated by reference. In the experiments whose results are presented below the NDV utilized was PPMK107 described in WO 2000/062735.

The virus can be incorporated in a pharmaceutical composition that comprises the virus and a pharmaceutically acceptable carrier. This invention provides a method for treating a neoplastic condition in a mammalian subject, comprising administering to the subject an amount of the virus described above effective to treat the neoplastic condition. For cancer treatment the virus can be administrated to the patients via any conventional route, for example by one or more intratumoral or intravenous injections. For intratumoral administration, the dose range can be from 1x10⁷ to 5x10¹² pfu/per tumor. For intravenous administration, the dose range can be from 1x10⁷ to 1x10¹³ pfu/m². ('Pfu' is an abbreviation for 'plaque forming unit'.)

The oncolytic virus according to this invention could also be engineered to incorporate other molecules such as GMCSF to enhance the efficacy of the oncolytic virus. In addition, the oncolytic virus could be a part of a combination cancer therapy with a checkpoint inhibitor such as anti-PD1 or anti-PDL1 molecule. Further, the oncolytic virus could be a part of a combination cancer therapy with other chemotherapeutic agents. The chemotherapeutic agents could be but are not limited to camptothecin compounds, for example, irinotecan or topotecan.

All publications, patents and patent applications mentioned in this specification are herein incorporated by reference in their entirety into the specification to the same extent as if each individual publication, patent or patent application was specifically and individually indicated to be incorporated herein by reference. Also incorporated by reference is any supplemental information that was published along with any of the aforementioned publications, patents and patent applications. For example, some journal articles are published with supplemental information that is typically available online.

The invention will be better understood by reference to the following examples, which illustrate but do not limit the invention described herein.

### EXAMPLES

### Example 1:

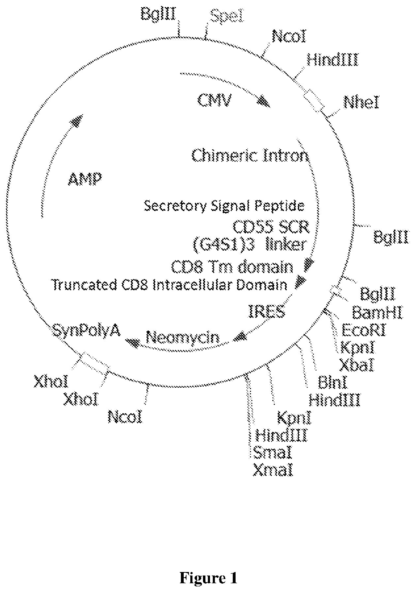

A modified version of recombinant CD55 with a four short consensus repeat (SCR) of CD55 downstream of the secretory signal peptide followed by a flexible linker (3xG4S1) and a CD8 transmembrane and a truncated CD8 intracellular domain was created (Figure 1). The coding sequence was cloned into a mammalian expression construct that has a CMV promoter, a synthetic intron driving the recombinant protein expression. The expression cassette also contained a drug selectable marker, neomycin phosphotransferase downstream from IRES. The gene expression cassette ended with a synthetic polyadenylation signal. SEQ ID NO:1 is the nucleotide sequence of the mammalian cell expression construct. SEQ ID NO:2 represents the amino acid sequence of the expressed protein. When expressed on chicken embryonic fibroblast DF1 cell surface or incorporated onto virus membrane, the signal peptide is cleaved yielding the mature recombinant fusion protein (SEQ ID NO:3) which has a configuration/orientation such that the CD55 SCR is on the outside of the cell or viral membrane, the flexible linker adjacent to the cell or viral membrane should provide maximal flexibility for the SCR of CD55 to exercise its biological function, i.e., disabling C3 convertase which is the central regulator of complement pathway. The flexible linker is followed by a CD8 transmembrane domain and a truncated CD8 intracellular domain.

### Example 2:

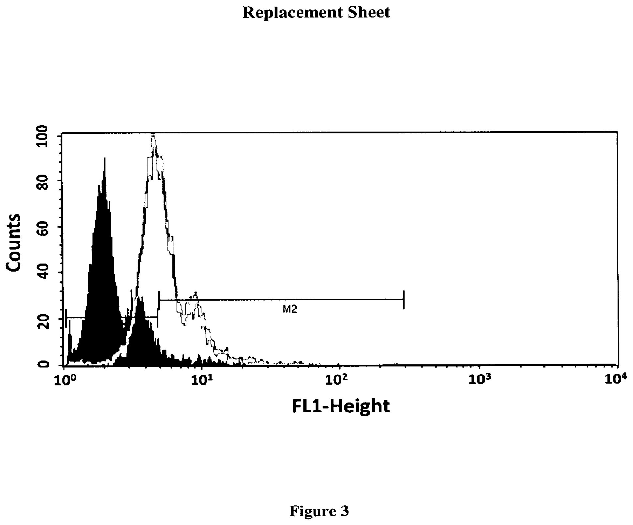

The mammalian expression construct was transfected into chicken embryonic fibroblast DF1 cells via PET 25K (polyethylenimine, linear 25 kDa, Polysciences, Cat. No. 23966) mediated transfection. Seventy two hours post-transfection, the transfected cells were selected in 300 µg/mL G418 (Geneticin^{®}, aminoglycoside antibiotic) to create a stable cell line that constitutively expresses SEQ ID NO:2. The stable cell line constitutively expressed SEQ ID NO:3 on its cell surface as detected by a monoclonal antibody (R&D Systems, Catalog No. MAB20091) that is specific for mature human CD55. As shown in Figure 3, the recombinant fusion protein was expressed on the DF1 cells stably transfected with the construct that encodes the recombinant fusion protein as analyzed by flow cytometry (Figure 3, the histogram on the right). The naive DF1 cells served as a negative control (Figure 3, the histogram on the left).

### Example 3:

The stable cell line expressing SEQ ID NO:3 on the cell surface was infected with wild type NDV that was produced from embryonated chicken eggs. The virus was then titered on human tumor cell line HT1080. Equal amount of virus (measured by PFU) was subjected to incubation with 40% normal human serum (NHS) and 40% heat-inactivated normal human serum (iNHS) respectively. The virus that remained alive after incubation with human serum was then scored on HT1080 cells by plaque assay. The ratio of virus recovered after incubation with NHS vs iNHS was calculated. As shown in Table 1, the recovery rate for the virus produced in embryonated chicken eggs was 0.5%, suggesting vast majority of the NDV particles produced by chicken eggs were inactivated most likely by human alternative complement pathway. Likewise, the recovery rate for the virus produced by the parental chicken embryonic fibroblast DF1 cells was 0.5%. Surprisingly, the recovery rate for the virus produced from the bulk non-clonal DF1 cells that stably expressed SEQ ID NO:3 on the cell surface was 5.8%, greater than 10 fold more than the wild type virus. When a total of 11 clonal populations of DF1 cells expressing SEQ ID NO:3 were examined, the recovery rate ranged from 0.8 to 20% with five clones scoring a lower recovery rate and six clones scoring a higher recovery rate than the bulk non-clonal cell line (Table 1). The virus generated by clone number 8 had a recovery rate of 10% which was 20 fold higher than the virus either produced by embryonated chicken eggs or the parental DF1 cells. The virus generated by clone number 40 had a recovery rate of 20% which was 40 fold higher than the virus either produced by embryonated chicken eggs or the parental DF1 cells. These data strongly suggest that the complement activity presented in normal human serum rapidly destroyed the NDV particles that were produced by embryonated chicken eggs or the parental chicken embryonic fibroblast DF1 cells. However, the new NDV particles produced by DF-1 cells that stably expressed recombinant complement inhibitor on the cell surface showed a remarkable up to 40-fold higher recovery rate as compared to the virus either produced by chicken eggs or the parental DF1 cells after incubation with 40% normal human serum under identical experimental conditions.

**Table 1. Virus recovery rate measured by the ratio of the virus recovered after incubation with 40% normal human serum (NHS) vs 40% heat-inactivated human serum (iNHS)**

| Oncolytic NDV Produced from | % Recovery rate after incubation with human serum |
|---|---|
| Embryonated Chicken Eggs | 0.5 |
| Parental DF1 Cells | 0.5 |
| Non-clonal DF1 cells expressing SEQ ID NO:3 | 5.8 |
| Clone #1 DF1 expressing SEQ ID NO:3 | 4.3 |
| Clone #2 DF1 expressing SEQ ID NO:3 | 5.2 |
| Clone #3 DF1 expressing SEQ ID NO:3 | 0.8 |
| Clone #4 DF1 expressing SEQ ID NO:3 | 6.8 |
| Clone #5 DF1 expressing SEQ ID NO:3 | 3.6 |
| Clone #6 DF1 expressing SEQ ID NO:3 | 1.5 |
| Clone #7 DF1 expressing SEQ ID NO:3 | 7.1 |
| Clone #8 DF1 expressing SEQ ID NO:3 | 10.0 |
| Clone #10 DF1 expressing SEQ ID NO:3 | 6.1 |
| Clone #11 DF1 expressing SEQ ID NO:3 | 6.0 |
| Clone #40 DF1 expressing SEQ ID NO:3 | 20.0 |

### Example 4:

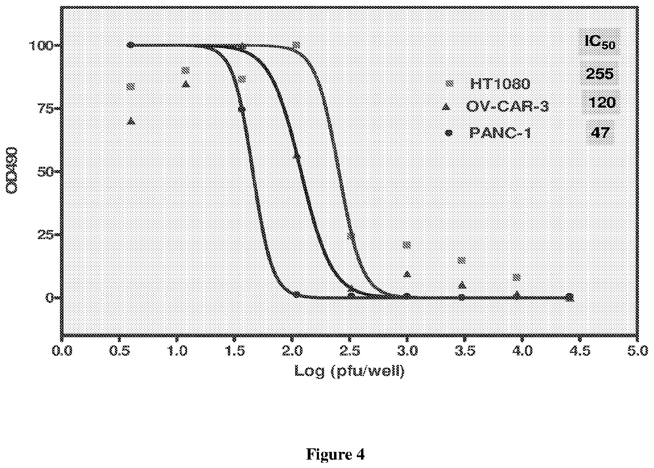

The broad spectrum oncolytic activity of NDV that was produced from the DF1 cells stably expressing the complement inhibitory fusion protein on their cell surface (Clone Number 8) was assessed using CellTiter96^{®} AQueous One Solution. This solution functions similar to MTT (i.e., 3-(4,5-dimethylthiazol-2-yl)-2,5-diphenyltetrazolium bromide) assays where metabolically active cells are able to bio-reduce MTS tetrazolium (i.e., 3-(4,5-dimethylthiazol-2-yl)-5-(3-carboxymethoxyphenyl)-2-(4-sulfophenyl)-2H-tetrazolium) in the reagent into soluble chromogenic formazan. Briefly, three different tumor cell lines HT1080 (fibrosarcoma), PANC-1 (pancreatic epithelial carcinoma) and OV-CAR3 (ovarian adenocarcinoma) where grown in separate 96 well plates. The following day, serial dilutions of the NDV virus were added to respective wells and the plate was incubated for 6 days at 37°C incubator with 5% CO2. On Day 6, the absorbance of all wells on each plate was measured at 490 nm using a spectrophotometer. IC₅₀ was calculated using 4 parameter logistic nonlinear regression analysis for each cell line. This resulted in final IC₅₀ values of 255, 120 and 47 pfu/well for HT1080, OV-CAR-3 and PANC-1 cell lines respectively (Figure 4). These results indicate that the NDV particles produced by DF-1 cells that stably expressed recombinant complement inhibitor on its cell surface retain the ability to lyse various tumor cell lines in a dose dependent manner.

### REFERENCES

Carroll, M. C., E. M. Alicot, P. J. Katzman, L. B. Klickstein, J. A. Smith, and D. T. Fearon. 1988. Organization of the genes encoding complement receptors type 1 and 2, decay-accelerating factor, and C4-binding protein in the RCA locus on human chromosome 1. J. Exp. Med. 167:1271.
Rey-Campos, J., P. Rubinstein, and S. Rodriguez de Cordoba. 1988. A physical map of the human regulator of complement activation gene cluster linking the complement genes CR1, CR2, DAF, and C4BP. J. Exp. Med. 167:664.
Lublin, D. M., and J. P. Atkinson. 1989. Decay-accelerating factor: biochemistry, molecular biology, and function. Annu. Rev. Immunol. 7:35. 5. Nakano, Y., K. Sumida, N. Kikuta, N. H. Miura, T. Tobe, and M. Tomita. 1992. Complete determination of disulfide bonds localized within the short consensus repeat units of decay accelerating factor (CD55 antigen). Biochim. Biophys. Acta 1116:235.
Censullo, P., and M.A. Davitz. 1994a. How GPI-anchored proteins turnover: or where do they go after arrival at the plasma membrane. Semin Immunol. 6:81.
Censullo, P., and M.A. Davitz. 1994b. The fate of GPI-anchored molecules. Braz J. Med. Biol. Res. 27:289
Morgan, B. P., and S. Meri. 1994. Membrane proteins that protect against complement lysis. Springer Semin. Immunopathol. 15:369.
Turner A.J. 1994. PIG-tailed membrane proteins. Essays Biochem. 28:113.
Kim D.D., and W.C. Song. 2006. Membrane complement regulatory proteins. Clin. Immunol. 118:127.
Pecora, A.L., Rizvi, N., Cohen, G.I., Meropol, N.J., Sterman, D., Marshall, J.L., Goldberg, S., Gross, P., O'Neil, J.D., Groene, W.S., Roberts, M.S., Rabin, H., Bamat, M.K., and R.M. Lorence. 2002. Phase I trial of intravenous administration of PV701, an oncolytic virus, in patients with advanced solid cancers. J. Clin. Oncol. 20:2251.
Laurie, S.A., Bell, J.C., Atkins, H.L., Roach, J., Bamat, M.K., O'Neil, J.D., Roberts, M.S., Groene, W.S., and R.M. Lorence. 2006. A phase 1 clinical study of intravenous administration of PV701, an oncolytic virus, using two-step desensitization. Clin. Cancer Res. 12:2555.
Lorence, R.M., Roberts, M.S., O'Neil, J.D., Groene, W.S., Miller, J.A., Mueller, S.N., and M.K. Bamat. 2007. Phase 1 clinical experience using intravenous administration of PV701, an oncolytic Newcastle disease virus. 7:157.
Biswas, M., Johnson, J.B., Kumar, S.R.P. Parks, G.D., and E. Subbiah. 2012. Incorporation of host complement regulatory proteins into Newcastle disease virus enhances complement evasion. J. Virol. 86:12708.
Rangaswamy, U.S., Cotter, C.R., Chang, X., Jin, H., and Z. Chen. 2016. CD55 is a key complement regulatory protein that counteracts complement-mediated inactivation of Newcastle disease virus. J. Gen. Virol. 97:1765.

### CLAUSES

1. A fusion protein comprising: (a) a CD55 peptide sequence, (b) a linker sequence C-terminal to the CD55 sequence, (c) a transmembrane domain C-terminal to the linker sequence, and (d) an intracellular domain C-terminal to the transmembrane domain; wherein the fusion protein does not contain a GPI anchor.
2. The protein of clause 1, wherein the CD55 peptide sequence is a human CD55 peptide sequence.
3. The protein of clause 1, wherein sequence (a) comprises four short consensus repeats (SCR) of CD55.
4. The protein of clause 1, wherein the linker is a (G4S1)3 linker.
5. The protein of clause 1, wherein transmembrane domain is a CD8 transmembrane domain.
6. The protein of clause 1, wherein the intracellular domain is a truncated CD8 intracellular domain.
7. The protein of clause 1, wherein peptide sequence (a) is covalently bonded to peptide sequence (b) by a single peptide bond.
8. The protein of clause 1, wherein peptide sequence (a) is covalently bonded to peptide sequence (b) by a spacer.
9. The protein of clause 1, wherein peptide sequence (b) is covalently bonded to peptide sequence (c) by a single peptide bond.
10. The protein of clause 1, wherein peptide sequence (b) is covalently bonded to peptide sequence (c) by a spacer.
11. The protein of clause 1, wherein peptide sequence (c) is covalently bonded to peptide sequence (d) by a single peptide bond.
12. The protein of clause 1, wherein peptide sequence (c) is covalently bonded to peptide sequence (d) by a spacer.
13. The protein of clause 1, wherein the fusion protein further comprises a secretory signal peptide N-terminal to sequence (a).
14. The protein of clause 13, wherein the secretory signal peptide is a secretory signal peptide of CD55.
15. The protein of clause 13, wherein the N-terminal secretory signal peptide is covalently bonded to sequence (a) by a single peptide.
16. The protein of clause 13, wherein the N-terminal secretory signal peptide is covalently bonded to sequence (a) by a spacer.
17. The protein of clause 1, having the sequence SEQ ID NO:2.
18. The protein of clause 1, having the sequence SEQ ID NO:3.
19. A nucleic acid encoding the protein of any one of clauses 1 through 18.
20. The nucleic acid of clause 19, wherein the nucleic acid is DNA.
21. The nucleic acid of clause 20, further comprising one or more introns.
22. The nucleic acid of clause 19, encoding a protein having the sequence SEQ ID NO:2.
23. The nucleic acid of clause 22, having the sequence SEQ ID NO:1.
24. An expression vector comprising the nucleic acid of any one of clauses 19 through 23, operatively linked to a control sequence.
25. A cell line stably expressing the protein of any one of clauses 1 through 18 on the cell surface.
26. The cell line of clause 25, wherein the cell line is a mammalian cell line.
27. The cell line of clause 25, wherein the cell line is a DF-1 chicken embryonic fibroblast cell line.
28. An enveloped virus incorporating the protein of any one of clauses 1 through 18 on the virus membrane.
29. The virus of clause 28, wherein the virus is an oncolytic virus.
30. The virus of clause 29, wherein the oncolytic virus is a Newcastle Disease Virus.
31. A pharmaceutical composition comprising the virus of clause 29 or 30 and a pharmaceutically acceptable carrier.
32. A method for treating a neoplastic condition in a mammalian subject, comprising administering to the subject an amount of the virus of clause 28 effective to treat the condition.
33. The method of clause 32, wherein the virus is administered intratumorally.
34. The method of clause 32, wherein the virus is administered intravenously.

## Claims

1. An enveloped virus incorporating a fusion protein on the virus membrane, the fusion protein comprising: (a) a CD55 peptide sequence, (b) a linker sequence C-terminal to the CD55 sequence, (c) a transmembrane domain C-terminal to the linker sequence, and (d) an intracellular domain C-terminal to the transmembrane domain; wherein the fusion protein does not contain a GPI anchor.

2. The enveloped virus of claim 1, wherein:
(i) the CD55 peptide sequence is a human CD55 peptide sequence;
(ii) the CD55 peptide sequence comprises four short consensus repeats (SCR) of CD55;
(iii) the linker is a (G4S1)3 linker;
(iv) the transmembrane domain is a CD8 transmembrane domain; and/or
(v) wherein the intracellular domain is a truncated CD8 intracellular domain.

3. The enveloped virus of claim 1 or claim 2, wherein:
(i) the peptide sequence (a) is covalently bonded to the linker sequence (b) by a single peptide bond or by a spacer;
(ii) the linker sequence (b) is covalently bonded to the transmembrane domain (c) by a single peptide bond or by a spacer; and/or
(iii) the transmembrane domain (c) is covalently bonded to the intracellular domain (d) by a single peptide bond or by a spacer.

4. The enveloped virus of claim 1, wherein the fusion protein further comprises a secretory signal peptide N-terminal to the peptide sequence (a); optionally wherein the secretory signal peptide is a secretory signal peptide of CD55; optionally wherein the N-terminal secretory signal peptide is covalently bonded to the peptide sequence (a) by a single peptide or by a spacer.

5. The enveloped virus of claim 1, wherein the fusion protein comprises the sequence SEQ ID NO:2 or SEQ ID NO: 3.

6. The enveloped virus of any one of claims 1 through 5, wherein the virus is an oncolytic virus.

7. The enveloped virus of claim 6, wherein the oncolytic virus is a Newcastle Disease Virus.

8. A cell line infected with the enveloped virus of any one of claims 1 through 7, wherein the cell line stably expresses the fusion protein on the cell surface.

9. The cell line of claim 8, wherein the cell line is a mammalian cell line, or wherein the cell line is a DF-1 chicken embryonic fibroblast cell line.

10. A pharmaceutical composition comprising the enveloped virus of any one of claims 1 through 7 and a pharmaceutically acceptable carrier.

11. The enveloped virus of any one of claims 1 through 7 for use as a medicament for shrinking a tumor in a mammalian subject, wherein the medicament comprises an amount of the virus effective to treat the condition in the subject.

12. The enveloped virus of any one of claims 1 through 7 for use in a method for treating a neoplastic condition in a mammalian subject, the method comprising administering to the subject an amount of the virus effective to treat the condition

13. The enveloped virus for use of claim 11, wherein the medicament is for intratumoral administration or intravenous administration.

14. The enveloped virus for use of claim 12, wherein the enveloped virus is administered intratumorally, or wherein the enveloped virus is administered intravenously.

15. The enveloped virus for use of claim 11, wherein the tumor is selected from fibrosarcoma, pancreatic epithelial carcinoma, or ovarian adenocarcinoma.
